# FASCICULE DE BREVET EUROPEEN

(11) **EP 0 670 740 B1**
(45) Date de publication et mention de la délivrance du brevet: **16.06.1999**
(21) Numéro de dépôt: 94900891.6
(22) Date de dépôt: 30.11.1993
(51) Int. Cl.: A61M 1/00

(54) **VALVE POUR LE TRAITEMENT DE L'HYDROCEPHALIE**
VENTIL ZUR BEHANDLUNG VON HYDROCEPHALUS
VALVE FOR THE TREATMENT OF HYDROCEPHALUS

(30) Priorité: 30.11.1992 FR 9214413
(43) Date de publication de la demande: 13.09.1995
(73) Titulaire: Heyer-Schulte Neurocare, L.P., Pleasant Prairie, WI 53158-0390 (US)
(72) Inventeur: Glories, Serge, 11570 Cazilhac (FR); Drevet, Jean-Baptiste, 75005 Paris (FR)
(74) Mandataire: Robert, Jean-Pierre
(86) Numéro de dépôt international: FR9301176
(87) Numéro de publication internationale: WO9412222

(56) Documents cités:
- EP-A- 0 233 325
- FR-A- 2 372 366
- US-A- 3 886 948
- US-A- 4 127 110
- US-A- 4 551 128
- US-A- 4 657 530
- US-A- 4 867 741

## Description

La présente invention concerne un dispositif de régulation et de contrôle de l'écoulement d'un liquide organique dans un circuit de drainage artificiel s'étendant entre un site de production et un site de résorption d'un sujet. L'une des principales applications de l'invention concerne le drainage du liquide céphalo-rachidien en cas d'hydrocéphalie par exemple que ce soit au niveau de la boîte crânienne ou au niveau lombaire.

L'hydrocéphalie est une maladie provoquée notamment par le blocage des voies naturelles de drainage du liquide céphalo-rachidien. Un traitement de cette maladie consiste à mettre en place une dérivation de l'écoulement du liquide depuis, soit les ventricules de la cavité crânienne, soit la base du rachis vers un site de résorption notamment le péritoine.

Cette dérivation comporte une tubulure avec une valve de régulation de l'écoulement, et l'ensemble est placé sous la peau du malade.

Il existe actuellement sur le marché plusieurs types de valves pour assurer cette fonction. Parmi les plus simples, on citera des valves du genre clapet unidirectionnel. Il s'agit alors de soupapes constituées par un tube entaillé dont les fentes s'élargissent lorsque la pression interne au tube surmonte la force élastique du tube lui-même tendant à fermer ces fentes (à laquelle s'ajoute ou se retranche la pression régnant dans la partie de tubulure située en aval de cette soupape). Il peut s'agir également d'un clapet appliqué sur un siège par un ressort calibré définissant un seuil de pression pour l'ouverture de la valve.

L'un des inconvénients majeurs de ces valves est leur absence de possibilité de réglage. Or, il est nécessaire d'adapter à chaque malade les caractéristiques de la valve en fonction des caractéristiques naturelles de son organisme, et de modifier les caractéristiques de la valve au cours du temps pour un même malade, en fonction de l'évolution de sa maladie. Le remplacement de la valve par une autre de caractéristiques différentes, tant au réglage initial qu'au cours de l'évolution de la maladie, implique une intervention chirurgicale puisque cette valve est implantée sous la peau du sujet.

Il a donc été développé des valves réglables. Celles-ci comprennent un ressort de rappel d'un clapet (bille) sur son siège dont on peut modifier le tarage par l'extérieur sans intervention chirurgicale. Un organe de manoeuvre est attelé au ressort et peut tourner dans le boîtier de la valve. Cet organe est sensible au champ magnétique et peut être magnétiquement accouplé au travers de la peau à un organe moteur pour le faire changer de position. Ces valves sont en général complexes, donc de volume et d'encombrement importants, d'une fiabilité discutable et déréglable lors des chocs. En outre, elles comportent des éléments métalliques qui nuisent à certaines opérations telles que la radiographie.

D'une manière générale, compte-tenu de la faible valeur des efforts mis en oeuvre dans ce type de valves, leur construction est très délicate. En outre, la régularité du fonctionnement est très aléatoire, car l'équilibre du clapet, lorsqu'un débit s'établit au travers de la valve, est très sensible aux variations de pression et de viscosité du liquide. De plus, la miniaturisation poussée de ce type de valves en fait des dispositifs fragiles et sujets à destruction.

Par ailleurs, les valves connues ont un fonctionnement qui est soumis à des paramètres qui varient dans des proportions importantes, notamment en fonction de la position du patient. Ainsi, en position debout il peut exister jusqu'à 400 mm de colonne d'eau de différence de pression entre les deux sites de production et de résorption alors qu'en position couchée cette différence peut être nulle. En outre, la pression absolue notamment dans les cavités ventriculaires varie selon que le patient est debout ou couché (elle augmente lorsqu'il s'allonge). En d'autres termes, vu de la valve, tout se passe comme si l'un des sites était à forte variation de pression tandis que l'autre est à faible variation de pression mais non négligeable par rapport à la variation importante. Comme les valves connues sont réglées à des faibles pressions d'ouverture (de l'ordre de 5O à 150 mm de colonne d'eau), selon la hauteur de colonne d'eau du circuit, la valve peut être bloquée lorsque le patient est couché, ouverte normalement lorsque le patient est assis mais provoquer un surdrainage important lorsque le patient est debout, origine de collapsus cérébral.

On connait par le document FR-A-2 372 366 une valve dont le fonctionnement n'est pas influencé par la valeur de le pression d'entrée. Cependant cette valve ne résoud pas les problèmes de surdrainage.

On connait également par le document FR-A- 2.655.535 une valve dont de fonctionnement est indépendant de la différence de pression existant entre l'amont et l'aval du clapet de régulation du débit de drainage ; cette valve est cependant de fabrication et de mise au point délicate en demandant beaucoup de précision au plan de sa réalisation et un choix de matériau extrêmement fin.

La présente invention entend remédier aux inconvénients des valves actuellement utilisées qui n'apportent pas de solution satisfaisante au problème du surdrainage et de la valve du document cité qui n'a pas été mise sur le marché du fait de la complexité de sa réalisation et de sa mise au point.

En outre, par sa conception, la valve de l'invention peut contenir un capteur grâce auquel il est possible de procéder au monitoring d'un patient en acquérant de manière permanente ou discrète, selon ce qu'exige son état, la valeur de la pression intracrânienne et ainsi de pouvoir intervenir rapidement avant l'apparition de conséquences irréversibles dues à une pression trop importante ou trop faible dans la boîte crânienne.

A cet effet, l'invention a donc pour objet un dispositif de régulation et de contrôle de l'écoulement d'un liquide organique entre un site de production et un site de résorption d'un sujet dans un circuit de drainage s étendant entre les deux sites l'un étant à faible variation de pression et l'autre à forte variation de pression, notamment en fonction de la position du sujet, conforme à la revendication 1.

Grâce à cette disposition de base qui offre à la pression du site à faible variation de pression une surface importante (celle de la paroi mobile de la chambre) il est possible de développer une force importante sur la membrane pour ainsi moduler l'effort exercé par l'organe élastique de rappel sur l'organe de réglage de la section de passage. Il est ainsi possible, à partir d'une valve, dont on a défini par construction un point de fonctionnement acceptable correspondant à une situation du patient, de régler les efforts mis en jeu pour que la valve adopte un autre point de fonctionnement acceptable pour une autre situation du patient. Les deux situations choisies sont la position couchée et debout du patient, si bien que la valve fonctionne également correctement, c'est-à-dire dans des conditions physiologiquement acceptables pour les situations intermédiaires.

Dans un mode préféré de réalisation de l'invention, le corps de valve possède une troisième chambre dans laquelle débouche l'autre partie du passage interne reliée à l'autre conduit, séparée de la seconde chambre par une paroi pourvue d'une ouverture entourée d'un siège de soupape, l'organe de réglage étant un clapet mobile par rapport au siège.

La paroi mobile, qui pourrait être un piston coulissant dans une ouverture cylindrique ménagée dans une partie du corps de valve délimitant la première chambre, comporte de préférence une partie centrale rigide pour son attelage avec la queue du clapet et une partie périphérique en forme de diaphragme annulaire.

Dans une variante de réalisation, le clapet est attelé directement à la partie rigide du diaphragme, les trois chambres étant superposées dans le corps de valve. Il sera cependant préféré une autre variante de réalisation permettant de fabriquer une valve moins épaisse dans laquelle le clapet est attelé à la partie rigide de la paroi mobile par l'intermédiaire d'un levier, la troisième chambre étant alors disposée dans le corps de la valve au niveau de la première chambre.

Il sera très avantageux de prévoir que l'organe élastique de rappel du clapet sur son siège soit une lame élastique de flexion logée dans la première chambre dont une face est en appui simple à ses deux extrémités sur des butées du corps de valve dont l'une est réglable en position, l'autre face étant en appui simple par sa partie médiane sur le centre de la partie rigide de la paroi mobile.

Dans une variante préférée de réalisation le clapet est situé du côté du siège exposé à la pression du site de résorption du liquide organique. Il possède ainsi une fonction anti-retour interdisant la contamination du site de production par le site de résorption. En outre, la première chambre sera remplie d'un liquide (pour éviter une diffusion gazeuse au travers des parois de la valve) et comportera une seconde paroi (ou partie de paroi) déformable soumise à la pression extérieure sous-cutanée. Comme par construction cette chambre constitue une référence, cette sensibilité à la pression sous-cutanée constitue une auto-compensation de fonctionnement de l'une des composantes de la pression agissant de l'autre côté de la membrane due à la pression sous-cutanée.

Par ailleurs, la structure de la valve selon l'invention présente l'avantage d'être adaptée à une réalisation simple d'un dispositif de mesure et de contrôle de la pression régnant dans le site à faible variation de pression.

En effet, la partie rigide de la paroi mobile possède, sur sa face tournée vers la première chambre, une saillie pour l'appui de la lame élastique dans laquelle est incorporé un composant électrique de caractéristique variable en fonction de la pression à laquelle il est soumis, ce composant étant couplé à un enroulement formant un circuit oscillant passif. En outre, l'enroulement est situé dans la première chambre avec son axe sensiblement perpendiculaire à la surface de la peau du patient lorsque la valve est implantée.

Il est alors possible à la valve de coopérer avec un capteur de mesure de la pression, cette valve jouant le rôle de l'élément passif d'un transpondeur, le capteur en étant l'élément actif et comportant à cet effet un circuit oscillant comprenant un enroulement et un composant électrique identique à celui que comporte la valve, soumis à aucune contrainte, émetteur d'un champ magnétique variable de fréquence de référence déterminée susceptible d'être placé à proximité de la valve et des moyens pour capter le champ magnétique émis par le circuit oscillant récepteur incorporé dans la valve, pour comparer la fréquence de référence à la fréquence de champ magnétique en réponse, en déduire la contrainte mécanique à laquelle est soumis le composant électrique du circuit oscillant de la valve, et convertir cette contrainte en valeur de la pression du liquide céphalo-rachidien.

On comprend tout l'intérêt d'un tel dispositif au plan de l'acquisition de données, de la surveillance continue d'un patient dans une phase critique de son état, et d'une manière générale pour le "monitoring" d'un patient au moment de l'implantation d'une valve afin de parvenir au meilleur réglage possible de ses paramètres de fonctionnement.

D'autres caractéristiques et avantages de l'invention ressortiront de la description donnée ci-après de quelques exemples de réalisation.

Il sera fait référence aux dessins annexés parmi lesquels :
- la figure 1 est une vue en coupe d'un premier schéma de l'invention pour l'explication de son fonctionnement.
- la figure 2 est une vue en coupe par un plan perpendiculaire à celui de la figure 1, d'une variante de réalisation préférée de l'invention représentée à titre schématique,
- les figures 3, 4, 5 et 6 sont des vues d'une réalisation industrielle de la valve selon l'invention, les figures 3 et 5 étant des vues en coupe selon respectivement les lignes III-III et V-V des figures 4 et 6, qui sont des vues extérieures de la valve selon l'invention,
- la figure 7 est une vue schématique en coupe d'une valve équipée de l'élément passif de transpondeur,
- la figure 8 illustre par un schéma un dispositif de mesure et de contrôle de la pression intracrânienne conforme à l'invention.

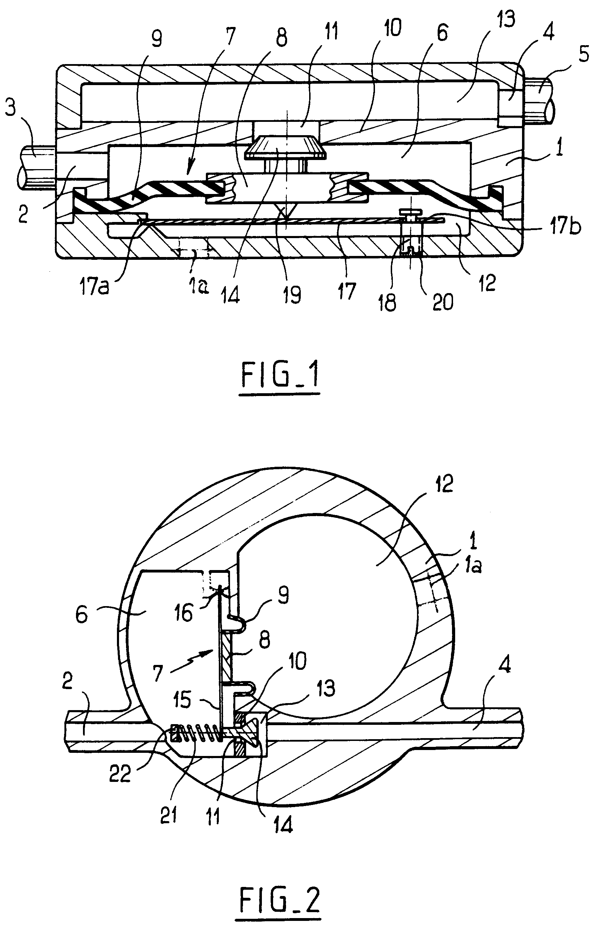

La vanne de l'invention représentée en coupe à la figure 1 comporte un corps 1 connecté par un orifice 2 à un conduit 3 et par un orifice 4 à un conduit 5. L'orifice 2 débouche dans une chambre 6 ménagée dans le corps 1 et comportant une paroi mobile 7. Cette paroi mobile comporte une partie centrale rigide 8 et un diaphragme souple déformable 9 annulaire reliant la partie centrale à la paroi latérale de la chambre 6. A l'opposé de la paroi mobile 7, la chambre 6 est limitée par une paroi 1O pourvue d'un orifice 11. La paroi mobile 7 délimite également avec le boîtier 1 une chambre 12 remplie d'un liquide à pression atmosphérique. La paroi du boîtier 1 qui limite la chambre 12, à l'opposé du diaphragme 7, comporte une partie souple 1a par laquelle la pression extérieure au boîtier se transmet à l'intérieur de la chambre 12.

L'orifice 11 ménagé dans la paroi 1O constitue une communication entre la chambre 6 et une troisième chambre 13 ménagée dans le corps de valve et de laquelle est issu le conduit 5. La paroi mobile 7, dans le cas de la figure 1, porte directement un clapet 14, capable d'obturer l'orifice 11 dont le bord constitue un siège pour le clapet 14 équipé de tout matériau apte à assurer une bonne assise du clapet dans une première position. Ce clapet 14 est solidaire de la partie rigide 8 de cette paroi mobile et sa position par rapport au siège définit la section de passage entre les chambres 6 et 13. La section utile de ce clapet est notable-ment inférieure aux surfaces de la paroi mobile exposées à la pression régnant dans les chambres 12 et 6 de sorte que l'influence de la pression régnant dans la chambre 13 sur le déplacement de la paroi mobile est réduite.

En outre la valve de l'invention comporte un organe élastique de rappel du clapet 14 contre son siège. Cet organe élastique est constitué par une lame de flexion élastique 17 logée dans la chambre 12, en appui simple (ou articulée) par l'une de ses extrémités 17a sur le boîtier et en appui simple par son autre extrémité 17b sur une butée réglable constituée par une vis 18 dont la tête est accessible par l'extérieur du boîtier. Par son autre face et en partie médiane, la lame 17 prend appui sur une saillie 19 que comporte, à l'opposé du clapet, la partie rigide 8 de la paroi mobile 7. Dans une variante non représentée, la saillie peut être remplacée par une bille logée dans un petit évidement de la lame 17 et prenant appui contre la partie rigide 8 du diaphragme 9.

La construction, le fonctionnement et le réglage de la valve s'opèrent de la manière suivante.

La mise en place du diaphragme 9 et du ressort 17 dans le boîtier 1, le remplissage de la chambre 12 sont tels qu'ils satisfont au premier test suivant : on introduit dans la valve par l'orifice 2 un débit fixe de liquide (par exemple 10 cm³/heure) et on règle le ressort 17 pour que le liquide entrant s'écoule depuis l'orifice 2 jusqu'à l'orifice 4 à la pression atmosphérique. On connecte ensuite à l'orifice 4 un conduit qui crée dans la chambre 13 une dépression d'environ 300 mm de colonne d'eau et on mesure la pression dans la chambre 6. Celle-ci doit être négative (dépression) d'une valeur définie (par exemple comprise entre 0 et -100 mm de colonne d'eau). Toute valve qui ne satisfait pas par construction à ce premier critère est refusée.

On procède ensuite au réglage consistant en agissant sur la vis 18 à ramener le clapet vers son siège de sorte que pour une pression dans la chambre 13 de l'ordre de moins 300 mm de colonne d'eau, la pression dans la chambre 6 soit pratiquement nulle (c'est-à-dire égale à la pression atmosphérique) le débit étant toujours de 10 cm³/heure. La valve est alors prête à être implantée dans une première application, c'est-à-dire sur un conduit de drainage s'étendant entre une cavité ventriculaire du cerveau (site de production) et par exemple un site péritonéal de résorption. Le fonctionnement de la valve implantée s'opère de la manière suivante. On supposera que cette valve est telle que la valeur définie au test de sa construction est de -50 mm de colonne d'eau, pour correspondre aux données physiologiques d'un patient qui, couché possède une pression intraventriculaire de 50 mm de colonne d'eau, et sensiblement zéro debout, avec un débit moyen de liquide céphalo-rachidien produit de 10 cm³/heure.

Ainsi le patient étant debout, la pression qui règne dans la chambre 6 est nulle, la pression dans la chambre 13 est de -300 mm de colonne d'eau et la valve débite 10 cm³/heure comme après son réglage.Les fluctuations de la pression intraventriculaire conduiront à un asservissement de la position du clapet 14 à ces pressions. Si celle-ci chute, le ressort 17 tend à repousser le clapet vers son siège, le débit est plus faible et la pression intraventriculaire remonte progressivement jusqu'à devenir positive. Dans ce cas la membrane tend à écarter le clapet de son siège, le débit augmente et la pression diminue.

Le patient étant ensuite couché, la pression intraventriculaire passe à 50 mm de colonne d'eau tandis que la pression dans la chambre 13 est pratiquement nulle. Dans ce cas, cette pression se traduit grâce au diaphragme 9 par un éloignement du clapet 14 de son siège, un accroissement de la section de passage suffisant pour que le débit soit maintenu constant au travers de l'orifice 11 alors que le différentiel de pression entre les chambres 6 et 13 est beaucoup plus faible que lorsque le patient était debout.

La valve selon l'invention est donc adaptée à un drainage correct sans surdrainage en position debout ni blocage en position couchée. On notera dans cette application que, vu de la valve, le site à grande variation de pression (300 mm de colonne d'eau) est le site de résorption tandis que le site à faible variation de pression (50 mm de colonne d'eau) est le site de production.

La valve représentée schématiquement à la figure 1 convient également, dans son principe, à une seconde application dite lombopéritonéale. Il s'agit alors de drainer le liquide céphalo-rachidien entre la zone lombaire du rachis (site de production) et une zone du péritoine (site de résorption).

Dans ce cas la valve doit satisfaire par construction au test suivant (avec par exemple les mêmes valeurs que précédemment) :
- un débit fixe est introduit à la pression atmosphérique dans la valve par l'orifice 4 et le ressort est placé de manière que le liquide ressorte par l'orifice 2 sans augmentation de pression à l'entrée 4.
- on connecte ensuite à l'orifice 4 un conduit qui crée une surpression d'environ 400 à 500 mm de colonne d'eau à l'entrée et on doit constater que la pression dans la chambre 6 est légèrement négative (de l'ordre de 50 à 100 mm de colonne d'eau) pour toujours le même débit.

Ensuite on agit sur la vis 18 pour que la pression dans la chambre 6 soit pratiquement nulle.

Une valve lombo-péritonéale ainsi construite fonctionne de la manière suivante. Le patient étant debout, la valve débite comme testée avec 400 à 500 mm de colonne d'eau en pression d'entrée. En position couchée, la pression dans la chambre 6 va remonter en éloignant le clapet 14 de son siège pour agrandir la section de passage du fluide qui est pression quasi nulle en entrée mais qui n'aura pas de pertes de charge à vaincre pour s'écouler naturellement.

Dans le cas d'une application lombopéritonéale le site de résorption est celui à faible variation de pression tandis que le site producteur de fluide est à forte variation de pression, vu de la valve.

A la figure 2 on retrouve certains des éléments déjà décrits avec les mêmes références. A propos de cette réalisation, on notera que le diaphragme 9 est fin et sans raideur élastique. La fixation de ce diaphragme est assurée par tout moyen approprié (collage, soudage aux ultrasons...) que ce soit sur les parois de la chambre ou à la partie centrale. Les surfaces de toutes les parois des chambres 6, 12 et 13 ainsi que celles des orifices 2 et 4 sont recouvertes de carbone au moyen d'un traitement connu mettant en oeuvre le craquage sous vide d'une atmosphère d'acétylène. Le clapet 14 est ici attelé à un levier 15 solidaire de la partie centrale rigide 8 de la paroi mobile 7, ce levier 15 étant en appui simple ou articulé sur le boîtier 1 par son extrémité 16 opposée à celle qui porte le clapet 14. La présence de ce levier présente un double intérêt. Tout d'abord il permet de disposer la chambre 13 au même niveau que la chambre 12, donc de réduire comme on le verra plus précisément dans les figures suivantes, la hauteur de la valve de l'invention par rapport à une réalisation conforme à la figure 1. En outre ce levier offre une démultiplication du mouvement de la paroi mobile 7 pour le clapet 14, ce qui permet d'avoir au niveau du siège de ce clapet, rapidement une section de passage importante. On notera également que le clapet 14 est, dans cette réalisation, monté de manière à avoir un effet de clapet anti-retour. On comprend en effet que contrairement au schéma de la figure 1 et pour une application dans laquelle le site de résorption est connecté à l'orifice 4, il s'ouvre dans le sens de l'écoulement; un écoulement inversé tendra donc à le plaquer sur son siège. Cet effet sera d'autant plus sensible que la liaison du levier 15 avec le clapet 14 peut être avec jeu le long du clapet.

A la figure 2 l'organe élastique de rappel du clapet 14 contre son siège est représenté sous forme d'un ressort de traction 21 logé quant à lui dans la chambre 6 et donc le réglage peut être réalisé par le déplacement de son point d'attelage 22 opposé à l'attelage de ce ressort au clapet 14. Les moyens de réglage de la position de cet élément d'attelage 22 ne sont pas représentés sur cette figure.

On remarquera sur cette figure également la présence de la portion de paroi déformable 1a qui permet de faire régner dans la chambre 12 une pression égale à celle sous-cutanée du patient. L'intérêt de cette disposition est d'annuler de la pression agissant sur la membrane dans la chambre 6 les variations uniquement dues aux variations de la pression sous-cutanée.

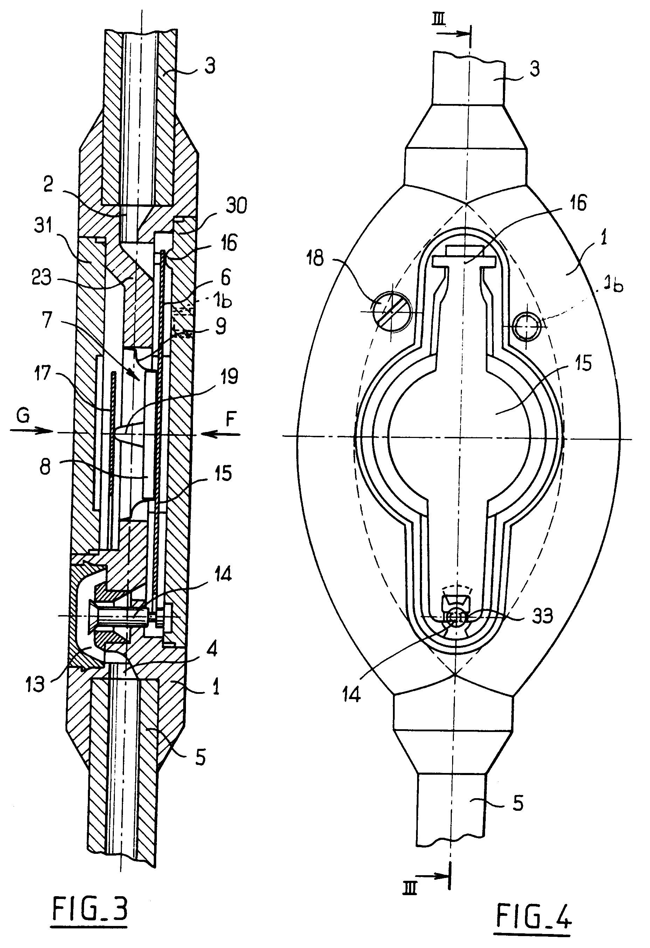

Aux figures 3 à 6, on a représenté une valve conforme à l'invention dans une configuration plus industrielle. On retrouve dans ces figures la plupart des éléments déjà décrits avec les mêmes références. Le corps 1 est ici un corps ovoïde allongé, en matériau synthétique biologiquement compatible, qui possède une paroi médiane 23 pourvue d'un orifice central fermé par la paroi mobile 7, cette paroi 23 séparant la chambre 6 de la chambre 12. Ces deux chambres 6 et 12 sont délimitées avec le corps 1 par des couvercles également en matériau biologiquement compatible respectivement 3O et 31, dont les faces internes sont également traitées pour être recouvertes de carbone. Les couvercles sont rapportés au corps par tout moyen. Le levier 15 pivote par son extrémité 16 sur un bossage 32 du couvercle 3O tandis que son autre extrémité est formée en une fourche 33 qui coopère avec la queue du clapet 14 pour l'appliquer sur son siège, sous l'effet de la lame de ressort 17 transmise par la saillie 19 et la partie rigide 8 de la paroi mobile au levier 15. La sensibilité de la valve sera améliorée si la liaison entre le levier 15 et le clapet 14 autorise un jeu le long de la queue du clapet. En effet en cas d'inversion de pression conduisant à un retour de fluide du site de résorption vers le site de production, le levier s'éloigne du siège et le clapet se' ferme sous l'effet d'un début de débit inverse. La figure 4 est un dessin de la valve vue suivant F de la figure 3, le couvercle 3O ayant été retiré.

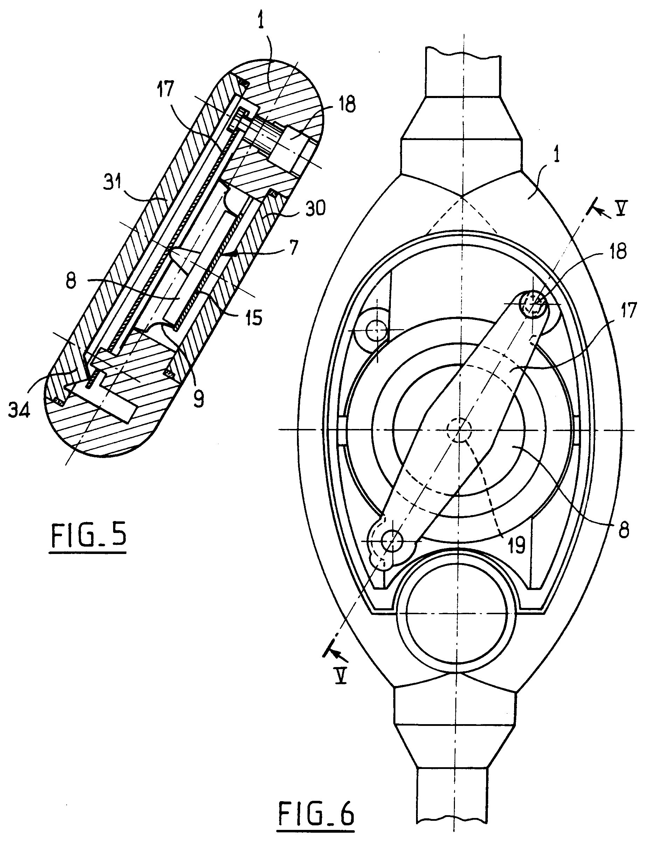

La figure 6 est une vue suivant G de la figure 3, le couvercle 31 ayant été enlevé. On voit que la lame de ressort flexible 17 est disposée en biais par rapport au levier 15, cette lame 17 coopérant avec la vis 18 et un bossage 34 du couvercle 31. Lorsque l'on dévisse la vis 18, on force la lame 17 en appui sur la saillie 19 solidaire de la partie rigide 8 de la paroi mobile 7. Au contraire, lorsque l'on enfonce la vis 18, on procède à un relâchement du ressort 17. La figure 5 est une coupe de la valve selon la ligne V-V de la figure 6 qui est la direction longitudinale de la lame de ressort 17.

On notera enfin aux figures 3 et 4 la forme de réalisation de la portion de paroi 1a à savoir un bouchon creux à parois fines 1b bouchant un orifice du corps 1.

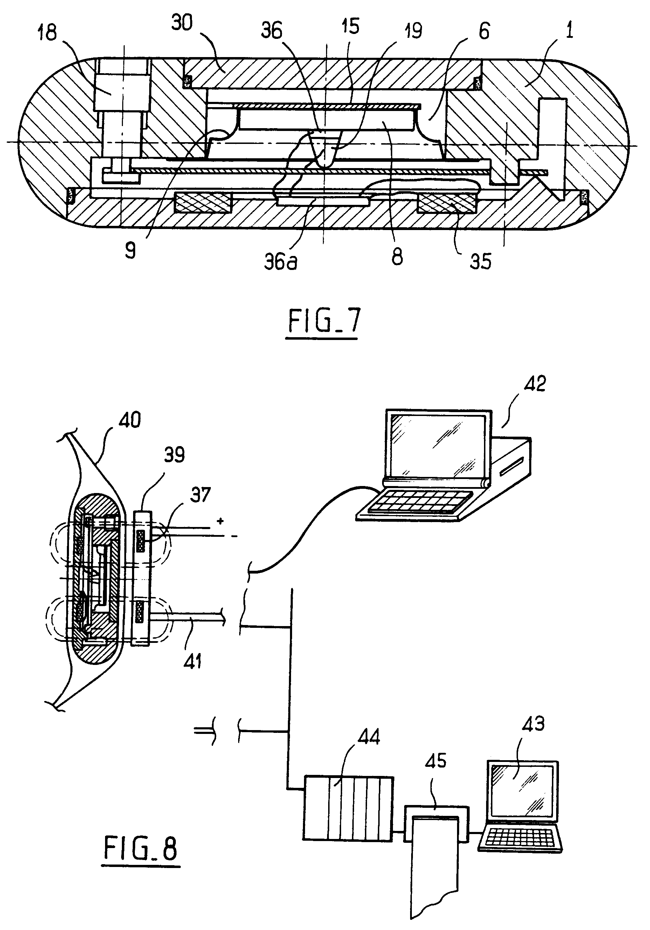

La figure 7 est une vue en coupe identique à celle de la figure 5 d'une variante de réalisation de l'invention qui permet à la valve de régulation du débit, d'être en plus un instrument de mesure et de contrôle de la pression du liquide céphalo-rachidien dans la boîte crânienne. Outre les éléments déjà décrits qui portent sur cette figure 7 les mêmes références, la valve de l'invention comporte à l'effet d'être un capteur de détection de la pression intracrânienne, un circuit électrique oscillant comportant un enroulement 35, un composant 36 ici constitué par un quartz intercalé entre la partie 8 rigide de la paroi mobile et la saillie 19 en appui sur la lame de ressort 17, dont la fréquence propre varie en fonction de la contrainte de compression qu'il subit et d'un quartz de référence 36a non contraint. Le composant 36 pourrait être constitué par, par exemple une jauge de contrainte disposée sur l'une des faces de la lame de ressort 17; il s'agirait alors d'un oscillateur à relaxation. On comprend que la caractéristique variable en fonction de la pression régnant dans la chambre 12 du composant 36 (quartz ou jauge de contrainte), modifie la fréquence de résonnance du circuit oscillant.

Le circuit oscillant intégré dans la valve de l'invention, constitue l'élément passif d'un transpondeur dont l'élément actif est constitué par un circuit oscillant d'excitation avec un bobinage 37. La figure 8 illustre cet élément actif rassemblé dans une électrode 39 que l'on peut placer au voisinage de la valve de l'invention lorsqu'elle est implantée sous la peau 4O d'un patient. L'excitation du circuit oscillant 37 engendre un champ magnétique de fréquence déterminée qui va créer dans le circuit oscillant passif de la valve un courant induit dont la fréquence sera fonction de la différence des états de contrainte des composants 36 et 36a. Le courant induit ainsi modulé crée en réponse un champ magnétique qui peut être capté par l'électrode 39 d'une fréquence de type battement, représentatif de l'état de contrainte du quartz 36. Pour qu'il y ait un maximum de couplage magnétique entre les circuits oscillants actif et passif, il faut que les bobinages soient parallèles donc que le bobinage 35 soit d'axe perpendiculaire à la peau du patient lorsque la valve est implantée.

Bien entendu les circuits oscillants actifs et passifs comportent des composants électroniques non décrits mais connus en eux-mêmes tels que des capacités et des transistors.

La sortie 41 de l'électrode 39 est celle qui recueille le battement résultant de la différence de fréquence entre le signal émetteur et le signal en réponse, cette différence de fréquence peut être transmise et traitée par un microprocesseur 42 au moyen duquel on peut suivre et contrôler l'évolution de la pression mesurée.

La figure 8 fait apparaître qu'un microprocesseur 43 associé à des mémoires 44 et à une imprimante 45 peut convenir pour la surveillance de plusieurs patients auxquels serait appliqué un capteur selon l'invention. On peut également connecter la sortie 41 du transpondeur à un appareil d'enregistrement portatif qui permettrait de contrôler et de vérifier le bon fonctionnement de la valve implantée.

L'organe d'obturation décrit ci-dessus sous la forme d'un clapet peut être constitué par tout moyen équivalent. En particulier, il peut consister en une bille sollicitée par deux ressorts en opposition, la lame de ressort 17 agissant par la partie rigide de la paroi mobile et par le levier 15 sur la bille dans la direction tendant à soulager le ressort dont l'effet est d'ouvrir le clapet.

L'invention trouve également une application dans le drainage artificiel d'autres fluides organiques tels que les ascites (sérosités dans le péritoine).

## Revendications

1. Dispositif de régulation et de contrôle de l'écoulement d'un liquide organique entre un site de production et un site de résorption d'un sujet dans un circuit de drainage s'étendant entre les deux sites l'un étant à faible variation de pression et l'autre à forte variation de pression, notamment en fonction de la position du sujet, le dispositif comprenant une valve à transplantation sous cutanée, reliée par un premier conduit à l'un des sites et par un second conduit à l'autre site, la valve comprenant un corps (1) délimitant un passage interne de liaison des deux conduits, un organe (14) de réglage de la section de ce passage de liaison et des moyens de commande (7, 15) accouplés à cet organe (14) de réglage pour le déplacer entre une première position de fermeture du passage et des secondes positions d'ouverture contrôlée de ce passage, ces moyens de commande comprenant une paroi mobile (7) ménageant dans le corps deux chambres (6, 12) l'une (6) formant une partie (2) du passage de liaison, l'autre étant fermée de manière étanche et des moyens d'attelage (15) de la paroi mobile (7) à l'organe de réglage (14), caractérisé en ce que :
- ladite chambre (12) fermée de manière étanche est à la pression extérieure au corps de valve,
- l'autre chambre (6) qui forme une partie (2) du passage interne est connectée au conduit (3) relié au site à faibles variations de pression et,
- l'organe de réglage (14) est attelé à des moyens élastiques (17, 21) de son rappel en direction de sa position de fermeture du passage de liaison qui comportent des moyens (18, 22) de réglage de l'intensité de l'effort de rappel qu'ils développent.

2. Dispositif selon la revendication 1, caractérisé en ce que le corps de valve (1) possède une troisième chambre (13) dans laquelle débouche l'autre partie (4) du passage interne reliée à l'autre conduit, séparée de la seconde chambre (6) par une paroi (10) pourvue d'une ouverture (11) entourée d'un siège de soupape, l'organe de réglage (14) étant un clapet mobile par rapport au siège.

3. Dispositif selon la revendication 2, caractérisé en ce que la paroi mobile (7) comporte une partie centrale rigide (8) pour son attelage avec la queue du clapet (14) et une partie périphérique souple (9) en forme de diaphragme annulaire.

4. Dispositif selon la revendication 3, caractérisé en ce que le clapet (14) est attelé directement à la partie rigide (8) de la paroi mobile (7), les trois chambres (12, 6, 13) étant superposées dans le corps (1) de valve.

5. Dispositif selon la revendication 3, caractérisé en ce que le clapet (14) est attelé à la partie rigide (8) de la paroi mobile (7) par l'intermédiaire d'un levier (15), la troisième chambre (13) étant disposée dans le corps de valve au niveau de la première chambre (12).

6. Dispositif selon l'une quelconque des revendications 3 à 5, caractérisé en ce que l'organe élastique de rappel (17) est logé dans la première chambre (12) et coopère avec la partie rigide (8) de la paroi mobile (7).

7. Dispositif selon la revendication 2, caractérisé en ce que le clapet (14) est situé du côté du siège exposé à la pression du site de résorption du liquide organique.

8. Dispositif selon l'une des revendications précédentes caractérisé en ce que la première chambre est remplie de liquide et comporte une seconde paroi déformable soumise à la pression extérieure.

9. Dispositif selon la revendication 6, caractérisé en ce que l'organe élastique (7) de rappel du clapet (14) sur son siège est une lame élastique (17) de flexion dont une face est en appui simple à ses deux extrémités sur des butées (34, 20) du corps (1) de valve, l'autre face étant en appui simple par sa partie médiane sur le centre (19) de la partie rigide (8) de la paroi mobile (7), l'une (20) des deux butées du corps (1) de valve est réglable en position perpendiculairement aux faces de la lame (17).

10. Dispositif selon la revendication 9, caractérisée en ce que la butée réglable est formée par l'extrémité d'une vis (18) coopérant avec un orifice taraudé du corps (1) faisant saillie dans la première chambre (12) et dont la tête est accessible et manoeuvrable par l'extérieur du corps (1) de valve (11).

11. Dispositif selon l'une des revendications 7 à 10, caractérisé en ce que la partie rigide (8) de la paroi mobile (7) possède, sur sa face tournée vers la première chambre (12), une saillie pour l'appui de la lame élastique (17) et en ce qu'il comporte, intégré à l'un des deux éléments, un composant électrique (36) de caractéristique variable en fonction de la pression à laquelle il est soumis, ce composant étant connecté à un enroulement (35) formant un circuit oscillant passif.

12. Dispositif selon la revendication 11 caractérisé en ce que l'enroulement (35) est situé dans la première chambre (12), son axe étant sensiblement perpendiculaire à la surface de la peau (40) du patient lorsque la valve est implantée.

13. Dispositif selon la revendication 11 ou la revendication 12, caractérisé en ce que le composant électrique (36) est un quartz intercalé entre la partie rigide (8) et la saillie (19).

14. Dispositif selon la revendication 11 ou la revendication 12, caractérisé en ce que le composant électrique est une jauge de contrainte placée sur la lame de ressort (17).

15. Dispositif de mesure de la pression régnant dans le site à faible variation de pression d'un patient équipé d'un dispositif de régulation selon l'une des revendications 13 à 14, caractérisé en ce qu'il comporte un circuit oscillant comprenant un enroulement (37) et un composant électrique (38) identique à celui que comporte la valve, soumis à aucune contrainte, émetteur d'un champ magnétique variable de fréquence de référence déterminée, susceptible d'être placé à proximité de la valve et des moyens pour capter le champ magnétique émis par le circuit oscillant récepteur (35, 36) incorporé dans la valve, pour comparer la fréquence de référence à la fréquence de champ magnétique en réponse, en déduire la contrainte mécanique à laquelle est soumis le composant électrique du circuit oscillant (35, 36) de la valve, et convertir cette contrainte en valeur de la pression du liquide céphalo-rachidien régnant dans la boîte crânienne.

16. Dispositif selon l'une des revendications précédentes caractérisé en ce que la surface intérieure des chambres (6,12,13) et des orifices (2,4) est recouverte de carbone.

## Claims

1. A device for regulating the flow of an organic liquid between a production site and a resorption site of a patient in a drainage circuit that extends between the two sites, one of the sites being a site of small pressure variation and the other being a site of large pressure variation, particularly as a function of the position of the subject, the device comprising a valve for implanting beneath the skin and connected by a first duct to one of the sites and by a second cut to the other site, the valve comprising a body (1) defining an internal passage interconnecting the two ducts, a member (14) for adjusting the section of said passage, and control means (7, 15) coupled to said adjustment member (14) to displace it between a first position in which the passage is closed and second positions in which the passage is opened to a controlled extent, said control means comprising a moving wall (7) forming in the body (1) two chambers (6, 12) one (6) of those chambers forming a portion (2) of said interconnecting passage and the other (12) of those chambers being closed in sealed manner, and means (15) for coupling the moving wall (7) to the adjustment member (14), characterized in that :
- said other chamber (12) closed in sealed manner is subjected to the pressure outside the valve body,
- said one chamber (6) which forms a portion (2) of the interconnecting passage is connected to the duct (3) connected to the site having small pressure variation and - said adjustment member (14) is connected to resilient means (17, 21) for returning the adjustment member towards its position in which it closes the interconnecting passage which comprise means (18, 22) for adjusting the magnitude of the return force.

2. A device according to claim 1, characterized the valve body (1) possesses a third chamber (13) into which the other portion (4) of the internal passage connected to the other duct opens out, which chamber is separated from the second chamber (6) by a wall (10) provided with an opening (11) surrounded by a valve seat, the adjustment member (14) being a valve member that is movable relative to the seat.

3. A device according to claim 2, characterized in that the moving wall (7) includes a rigid central portion (8) for coupling to the tail of the valve member (14) and a flexible peripheral portion (9) in the form of an annular diaphragm.

4. A device according to claim 3, characterized in that the valve member (14) is directly coupled to the rigid portion (8) of the moving wall (7), the three chambers (12, 6, 13) being superposed inside the body (1) of the valve.

5. A device according to claim 3, characterized in that the valve member (14) is coupled to the rigid portion (8) of the moving wall (7) by means of a lever (15), the third chamber (13) being disposed in the valve body at the same level as the first chamber (12).

6. A device according to any one of claims 3 to 5, characterized in that the resilient return member (17) is housed in the first chamber (12) and co-operates with the rigid portion (8) of the moving wall (7).

7. A device according to claim 2, characterized in that the valve member (14) is situated on the side of the seat which is exposed to pressure from the organic liquid resorption site.

8. A device according to any preceding claim, characterized in that the first chamber is filled with liquid and includes a second deformable wall subjected to the outside pressure.

9. A device according to claim 6, characterized in that the resilient member (7) for returning the valve (14) against its seat is a resilient flexible blade (17) having one face bearing in simple manner at both ends against abutments (34, 20) of the valve body (1), with the middle portion of its other face bearing in simple manner against the center (19) of the rigid portion (8) of the moving wall (7), one (20) of the two abutments of the valve body (1) being adjustable in position perpendicularly to the faces of the blade (17).

10. A device according to claim 9, characterized in that the adjustable abutment is formed by the end of a screw (18) co-operating with a tapped orifice in the body (1) and projecting into the first chamber (12), the head of the screw being accessible and drivable from outside the body (1) of the valve (11).

11. A device according to any one of claims 7 to 10, characterized in that the rigid portion (8) of the moving wall (7) possesses, on its face facing towards the first chamber (12), a projection against which the resilient blade (17) bears, and in that it includes, integrated in one of those two elements, an electrical component (36) having a characteristic that varies as a function of the pressure to which it is subjected, said component being connected to a winding (35) forming a passive oscillating circuit.

12. A device according to claim 11, characterized in that the winding (35) is situated in the first chamber (12), its axis being substantially perpendicular to the surface of the skin (40) of the patient when the valve is implanted.

13. A device according to claim 11 or claim 12, characterized in that the electrical component (36) is a quartz crystal interposed between the rigid portion (8) and the projection (19).

14. A device according to claim 11 or claim 12, characterized in that the electrical component is a strain gauge placed on the spring blade (17).

15. A device for measuring the pressure that obtains in the site having small pressure variation of a patient fitted with a regulator device according to any one of claims 11 to 14, the device being characterized in that it includes an oscillating circuit comprising a winding (37) and an electrical component (38) identical to the component included in the valve, and subjected to no stress, serving to emit a variable magnetic field of determined reference frequency and suitable for being placed in the proximity of the valve, and means for picking up the magnetic field emitted by the receiver oscillating circuit (35, 36) incorporated in the valve, for comparing the reference frequency with the frequency of the response magnetic field, for deducing therefrom the mechanical stress to which the electrical component of the oscillating circuit (35, 36) in the valve is subjected, and for conferring said stress into a value for the pressure of cerebrospinal fluid that obtains inside the cranium.

16. A device according to any preceding claim, characterized in that the inside surfaces of the chambers (6, 12, 13) and of the orifices (2, 4) is covered in carbon.

## Patentansprüche

1. Vorrichtung zum Regeln und Steuern des Fließens einer organischen Flüssigkeit zwischen einer Erzeugungsstelle und einer Resorptionsstelle einer Testperson in einem Drainagekreislauf, der sich zwischen den beiden Stellen erstreckt, wobei die eine einer geringen Druckschwankung und die andere einer starken Druckschwankung ausgesetzt ist, insbesondere in Abhängigkeit der Lage der Testperson, wobei die Vorrichtung ein zur Transplantation unter die Haut bestimmtes Ventil hat, das über einen ersten Kanal mit einer der Stellen und über einen zweiten Kanal mit der anderen Stelle verbunden ist, wobei das Ventil ein Gehäuse (1), das einen inneren Verbindungsdurchgang zum Verbinden der beiden Kanäle abgrenzt, ein Regelorgan (14) zum Regeln des Querschnittes dieses Verbindungsdurchgangs und Steuermittel (7, 15) hat, die an diesem Regelorgan (14) angekoppelt sind, um dieses zwischen einer ersten Stellung eines Verschließens des Durchgangs und zweiten Stellungen eines gesteuerten Öffnens dieses Durchgangs zu versetzen, wobei diese Steuermittel eine bewegliche Wand (7) haben, die in dem Gehäuse zwei Kammern (6, 12) ausbildet, von denen die eine (6) einen Abschnitt (2) des Verbindungsdurchgangs bildet und die andere luftdicht verschlossen ist, und Kopplungsmittel (15) zum Koppeln der beweglichen Wand (7) an das Regelorgan (14) haben, dadurch **gekennzeichnet**, daß
die luftdicht verschlossene Kammer (12) den außerhalb des Ventilgehäuses herrschenden Außendruck hat,
die andere Kammer (6), die einen Abschnitt (2) des inneren Durchgangs bildet, mit dem Kanal (3) verbunden ist, der mit der Stelle mit geringen Druckschwankungen verbunden ist, und
das Regelorgan (14) mit elastischen Mitteln (17, 21) zu seinem Rückstellen in Richtung seiner Stellung des Verschließens des Verbindungsdurchgangs gekoppelt ist, die Mittel (18, 22) zum Steuern der Größe der Rückstellkraft haben, die sie entwickeln.

2. Vorrichtung nach Anspruch 1, dadurch **gekennzeichnet**, daß das Ventilgehäuse (1) eine dritte Kammer (13) hat, in die der andere Abschnitt (4) des inneren Durchgangs mündet, der mit dem anderen Kanal verbunden ist, und die von der zweiten Kammer (6) durch eine Wand (10) getrennt ist, die mit einer Öffnung (11) versehen ist, die von einem Ventilsitz umgeben ist, wobei das Regelorgan (14) ein relativ zu dem Ventilsitz beweglicher Ventilkörper ist.

3. Vorrichtung nach Anspruch 2, dadurch **gekennzeichnet**, daß die bewegliche Wand (7) einen starren zentralen Abschnitt (8) zu ihrem Ankoppeln an den Schaft des Ventilkörpers (14) und einen biegsamen Randabschnitt (9) in Form einer ringförmigen Membran hat.

4. Vorrichtung nach Anspruch 3, dadurch **gekennzeichnet**, daß der Ventilkörper (14) direkt an den starren Abschnitt (8) der beweglichen Wand (7) angekoppelt ist, wobei die drei Kammern (12, 6, 13) in dem Ventilgehäuse (1) übereinander angeordnet sind.

5. Vorrichtung nach Anspruch 3, dadurch **gekennzeichnet**, daß der Ventilkörper (14) mittels eines Hebels (15) an den starren Abschnitt (8) der beweglichen Wand (7) angekoppelt ist, wobei die dritte Kammer (13) in dem Ventilgehäuse in Höhe der ersten Kammer (12) angeordnet ist.

6. Vorrichtung nach einem der Ansprüche 3 bis 5, dadurch **gekennzeichnet**, daß das elastische Rückstellorgan (17) in der ersten Kammer (12) untergebracht ist und mit dem starren Abschnitt (8) der beweglichen Wand (7) zusammenarbeitet.

7. Vorrichtung nach Anspruch 2, dadurch **gekennzeichnet**, daß der Ventilkörper (14) sich auf der Seite des Ventilsitzes befindet, die dem Druck der Resorptionsstelle der organischen Flüssigkeit ausgesetzt ist.

8. Vorrichtung nach einem der vorhergehenden Ansprüche, dadurch **gekennzeichnet**, daß die erste Kammer mit Flüssigkeit gefüllt ist und eine zweite verformbare Wand hat, die dem Außendruck ausgesetzt ist.

9. Vorrichtung nach Anspruch 6, dadurch **gekennzeichnet**, daß das elastische Rückstellorgan (7) zum Rückstellen des Ventilkörpers (14) auf seinen Ventilsitz eine biegeelastische Lamelle (17) ist, deren eine Seite mit ihren beiden Enden auf Anschlägen (34, 20) des Ventilgehäuses (1) einfach aufliegt, und deren andere Seite über ihren Mittelabschnitt an der Mitte (19) des starren Abschnittes (8) der beweglichen Wand (7) anliegt, wobei einer (20) der beiden Anschläge des Ventilgehäuses (1) senkrecht zu den Seiten der Lamelle (17) in seiner Lage verstellbar ist.

10. Vorrichtung nach Anspruch 9, dadurch **gekennzeichnet**, daß der verstellbare Anschlag durch das Ende einer Schraube (18) gebildet ist, die mit einer mit einem Gewinde versehenen Öffnung des Gehäuses (1) zusammenwirkt, in die erste Kammer (12) vorspringt und deren Kopf von außerhalb des Gehäuses (1) des Ventils (11) zugänglich und steuerbar ist.

11. Vorrichtung nach einem der Ansprüche 7 bis 10, dadurch **gekennzeichnet**, daß der starre Abschnitt (8) der beweglichen Wand (7) auf seiner in Richtung der ersten Kammer (12) zugewandten Seite einen Vorsprung zum Stützen der elastischen Lamelle (17) hat, und daß sie, integriert in einem der beiden Elemente, eine elektrische Komponente (36) hat, die in Abhängigkeit von dem Druck, dem sie ausgesetzt ist, veränderlich ist, wobei diese Komponente an eine Wicklung (35) angeschlossen ist, die einen passiven Schwingkreis bildet.

12. Vorrichtung nach Anspruch 11, dadurch **gekennzeichnet**, daß sich die Wicklung (35) in der ersten Kammer (12) befindet, wobei ihre Achse im wesentlichen senkrecht zur Oberfläche der Haut (40) des Patienten ist, wenn das Ventil implantiert ist.

13. Vorrichtung nach Anspruch 11 oder Anspruch 12, dadurch **gekennzeichnet**, daß die elektrische Komponente (36) ein Quarz ist, der zwischen dem festen Abschnitt (8) und dem Vorsprung (19) eingeschoben ist.

14. Vorrichtung nach Anspruch 11 oder Anspruch 12, dadurch **gekennzeichnet**, daß die elektrische Komponente ein Dehnungsmeßstreifen ist, der auf der Federlamelle (17) angeordnet ist.

15. Vorrichtung zum Messen des Druckes, der in der Stelle eines Patienten, die geringe Druckschwankungen hat, herrscht, wobei der Patient mit einer Regelvorrichtung nach einem der Ansprüche 13 bis 14 ausgestattet ist, **gekennzeichnet** durch einen Schwingkreis, der eine Wicklung (37) und eine elektrische Komponente (38) hat, die identisch zu der ist, die das Ventil hat, keiner mechanischen Spannung ausgesetzt ist, ein Sender eines veränderlichen Magnetfeldes mit vorgegebener Bezugsfrequenz ist, und in der Nähe des Ventils angeordnet werden kann, und Mittel zum Erfassen des Magnetfeldes, das durch den Empfangsschwingkreis (35, 36), der in dem Ventil aufgenommen ist, abgegeben wird, um die Bezugsfrequenz mit der Frequenz des als Antwort erzeugten Magnetfeldes zu vergleichen, daraus die mechanische Spannung, der die elektrische Komponente des Schwingkreises (35, 36) des Ventils ausgesetzt ist, abzuleiten, und diese Spannung in einen Wert des Druckes der Gehirn-Rückenmarksflüssigkeit, die in dem Gehirnschädel herrscht, umzuwandeln.

16. Vorrichtung nach einem der vorhergehenden Ansprüche, dadurch **gekennzeichnet**, daß die Innenfläche der Kammern (6, 12, 13) und der Öffnungen (2, 4) mit Kohlenstoff überzogen ist.
